# FASCICULE DE BREVET EUROPEEN

(11) **EP 1 205 214 B1**
(45) Date de publication et mention de la délivrance du brevet: **31.08.2011**
(21) Numéro de dépôt: 01402851.8
(22) Date de dépôt: 05.11.2001
(51) Int. Cl.: A61N 1/37, A61N 1/368

(54) **Dispositif médical actif implantable, notamment stimulateur cardiaque, défibrillateur, cardioverteur ou dispositif multisite, comprenant des moyens de détection d'un risque de situation de fusion**
Aktive implantierbare medizinische Vorrichtung, wie Herzschrittmacher, Entflimmerer oder Mehrstelleneinrichtung mit Mitteln zur Erfassung des Risikos eines Zusammenfalls der Herzkammerreizungpulsen und Klopfen
Active implantable medical device, of the type pacemaker, defibrillator, cardioverter or multisite device with means for detecting a risk of fusion

(30) Priorité: 08.11.2000 FR 0014294
(43) Date de publication de la demande: 15.05.2002
(73) Titulaire: ELA MEDICAL, 92541 Montrouge (FR)
(72) Inventeur: Casset, Cyril, 75010 Paris (FR); Limousin, Marcel, 75014 Paris (FR)
(74) Mandataire: Dupuis-Latour, Dominique

(56) Documents cités:
- EP-A- 0 597 728
- WO-A-93/02741
- US-A- 5 534 016
- US-A- 5 626 620
- US-A- 5 713 930
- US-A- 5 861 007

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, dispositifs "multisite", défibrillateurs et/ou cardioverteurs, tous dispositifs dont le fonctionnement dépend de la détection des signaux cardiaques spontanément produits par le coeur du patient porteur du dispositif pour enregistrer des données, poser un diagnostic ou appliquer une thérapie appropriée, notamment en délivrant au coeur des impulsions électriques de faible énergie pour le traitement des troubles du rythme cardiaque.

Elle concerne plus particulièrement les prothèses de type "double chambre", c'est-à-dire dans lesquelles il est possible de stimuler à la fois l'oreillette droite et le ventricule droit (configuration dite DDD). Elle s'applique bien évidemment aussi aux prothèses de type "triple chambre" (stimulation atriale droite et double stimulation ventriculaire) et "quadruple chambre" (double stimulation atriale et double stimulation ventriculaire) et, de façon générale, aux prothèses dites "multisite", dès lors que ces prothèses comportent au moins un site auriculaire et un site ventriculaire situés d'un même côté du coeur.

Le pilotage de la stimulation implique l'ajustement permanent de divers paramètres dont les principaux sont la fréquence de stimulation et le délai atrio-ventriculaire (DAV).

Ces paramètres, ainsi que la décision de stimuler ou non un ventricule, mettent en oeuvre le contrôle de présence ou d'absence d'un rythme ventriculaire spontané, associé à un rythme auriculaire (qui peut lui-même être spontané ou stimulé).

Un suivi clinique des patients a révélé que, dans différents cas, l'algorithme de pilotage du stimulateur se fait parfois leurrer par la survenue de "fusions", c'est-à-dire de stimulations intervenant de façon concomitante à une dépolarisation ventriculaire spontanée.

En effet, après une stimulation ventriculaire, l'événement ventriculaire détecté (complexe QRS) peut être soit le résultat direct de cette stimulation compte tenu du temps de latence existant entre ces deux événements, soit un complexe spontané survenant dans la même fenêtre temporelle (fusion).

La survenue d'une fusion peut avoir un effet délétère du point de vue hémbdynamique, du fait de la présence de deux contractions très rapprochées dont l'une est inutile.

On connaît divers dispositifs susceptibles de détecter des situations de fusion, comme par exemple ceux décrits dans les US-A-5 534 016 et US-A-5 626 620, ou encore dans le EP-A-0 597 728, qui prévient de faire varier le DAV de manière à ajuster celui ci à une valeur permettant d'éviter les conséquences délétères de la survenue d'une fusion. Pour cela, lorsque le DAV est tel qu'il cause une fusion, sa valeur est réduite au cycle suivant, de manière à pouvoir stimuler avec un DAV suffisamment long pour qu'une réponse évoquée puisse éventuellement être détectée, donc avec une valeur proche d'une fusion tout en évitant un risque de fusion.

Un autre paramètre régulièrement réajusté est le niveau de tension de l'impulsion de stimulation des cavités cardiaques, ventriculaires ou auriculaires, car le seuil de tension de stimulation, appelé "seuil d'entraînement", permettant de produire avec certitude une dépolarisation de la cavité myocardique, est une grandeur qui peut varier dans le temps. Il est donc souhaitable de pouvoir réévaluer à intervalles réguliers le niveau de l'amplitude de stimulation en opérant un test du seuil d'efficacité de la stimulation, appelé "test de capture".

Un algorithme de test automatique du seuil de capture ventriculaire est décrit par exemple dans le WO-A-93/02741 (Ela Médical), algorithme qui est mis en oeuvre dans le stimulateur de type *Talent* d'Ela Médical. Cet algorithme de test utilise en particulier la détection de présence ou d'absence d'un rythme ventriculaire spontané associé à un rythme auriculaire concomitant, spontané ou stimulé.

Un suivi clinique des patients a révélé que, dans différents cas, l'algorithme de test de capture se fait parfois leurrer par la survenue de "fusions".

Dans le cas d'un test de capture, même si une fusion n'a pas d'effet hémodynamique, elle est néanmoins susceptible de produire une surélévation de la valeur du seuil d'entraînement par rapport au seuil réel du patient, avec pour conséquence un réajustement de l'amplitude de simulation à un niveau excessif, maintenu au moins pendant plusieurs heures ; bien qu'il ne soit pas en lui-même dangereux, un tel niveau excessif constitue une source de surconsommation et donc de réduction de la durée de vie de l'implant. le US 5 713 930 A décrit un dispositif muni de moyens de test de capture et de moyens de détection de fusion analysant la variation du segment ST. En cas de fusion détectée, il est mis fin au test de capture.

L'un des buts de l'invention est de remédier à ces divers inconvénients en proposant un dispositif pourvu de moyens de détection d'un risque de fusion aptes à permettre à l'algorithme de pilotage de prendre toute action appropriée, par exemple d'inhiber la reprogrammation des paramétres de fonctionnement du stimulateur et de réitérer par exemple le test de capture à un moment ultérieur, après disparition de ce risque de fusion.

Pour atteindre ce but, l'invention propose un dispositif du type divulgué par le US 5 713 930 A précité, correspondant au préambule de la revendication 1. L'invention est définie par les éléments énoncés à la partie caractérisante de la revendication 1. Les sous-revendications visent des mises en oeuvre particulières, avantageuses.

On va maintenant décrire un exemple de mise en oeuvre de l'invention, en référence aux dessins annexés.
La figure 1 est un chronogramme montrant trois cycles cardiaques successifs modifiés de manière à permettre la détection d'une fusion éventuelle.
La figure 2 est un organigramme montrant le détail des différentes étapes de mise en oeuvre de cette détection.
La figure 3 est un chronogramme montrant trois cycles cardiaques successifs modifiés de manière à permettre la détection d'une fusion éventuelle, selon l'invention.
La figure 4 est un organigramme montrant le détail des différentes étapes de mise en oeuvre de ce mode de réalisation.

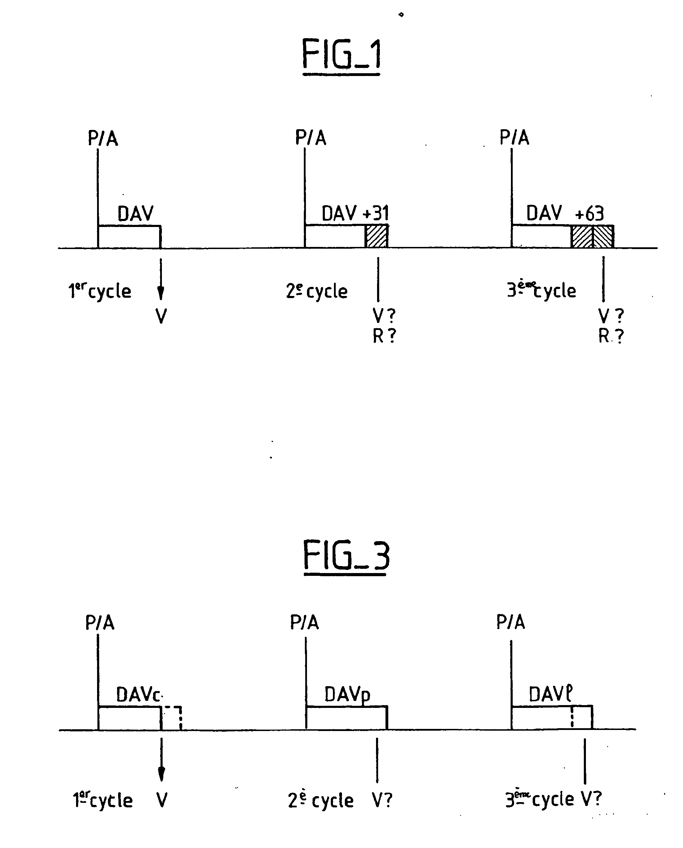

Sur la figure 1, on a représenté trois cycles cardiaques successifs, où les actions seront synchronisées sur un événement auriculaire, qu'il soit spontané (détection auriculaire P) ou stimulé (stimulation auriculaire A). Initialement, le délai atrio-ventriculaire (DAV) est fixé à sa valeur programmée par le médecin.

Dans l'exemple que l'on va décrire, il s'agit de choisir une durée de DAV utilisable pour la fonction de détermination automatique du seuil d'entraînement comme décrit par exemple dans le WO-A-93/02741.

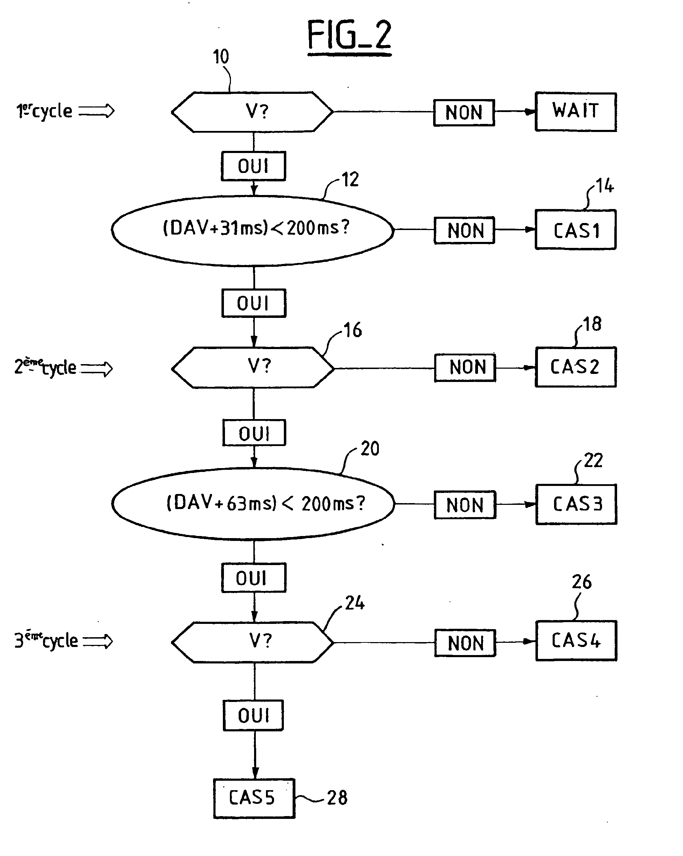

La première étape 10 de l'algorithme illustré figure 2 consiste à déterminer si l'on est bien en présence d'une situation où le ventricule doit être stimulé (stimulation ventriculaire V) à la fin du DAV programmé.

Si tel est bien le cas, l'algorithme allonge progressivement le DAV au cours des cycles suivants, pas à pas à chaque cycle. L'allongement est par exemple de 31 ms, dans la limite maximale permise pour la variation du DAV, par exemple dans une limite de 200 ms (étape 12). Mais la limite supérieure du DAV peut avantageusement être fonction de la fréquence cardiaque à laquelle le test est effectué.

Dans le premier cas, c'est-à-dire où l'allongement du DAV dépasserait la limite maximale permise (cas 1, étape 14), le DAV choisi est trop long et ne permet pas de contrôler la présence d'un rythme spontané. Par sécurité, le DAV est alors réduit, à une valeur DAV-63 ms.

Si, en revanche, l'allongement du DAV ne dépasse pas la limite maximale fixée, l'algorithme détecte alors, au cours d'un deuxième cycle, la survenue d'un événement ventriculaire stimulé (stimulation ventriculaire V) ou spontané (détection ventriculaire R) avant la fin du DAV allongé de 31 ms (étape 16).

La détection d'un événement ventriculaire spontané (cas 2, étape 18) révèle un risque de fusion pour le DAV programmé. Le DAV est donc fixé à une valeur courte, de préférence la valeur minimale, et satisfaisant la contrainte d'être inférieure à au moins 60 ms du DAV programmé.

En cas de survenue au deuxième cycle d'un événement ventriculaire stimulé, le DAV est une nouvelle fois rallongé, par exemple de 63 ms par rapport à sa valeur programmée, toujours dans la limite de la valeur maximale permise (étape 20).

S'il n'est pas possible d'augmenter le DAV sans dépasser cette limite maximale (cas 3, étape 22), pour tenir compte du risque de survenue d'un événement ventriculaire spontané en fin de DAV (entre DAV+31 ms et DAV+63 ms), le DAV est fixé à une valeur réduite par rapport à la valeur programmée, typiquement à DAV-31 ms.

Si le DAV a pu être allongé de 63 ms au troisième cycle, l'algorithme teste la survenue éventuelle d'un événement ventriculaire stimulé avant la fin du DAV allongé de 63 ms (étape 24).

L'absence d'un tel événement stimulé (cas 4, étape 26) révèle un risque de fusion. Aussi, comme dans le cas précédent, le DAV est réduit par rapport à sa valeur programmée, typiquement à DAV-31 ms.

Dans le cas contraire, c'est-à-dire en l'absence de détection dans les 63 ms suivant le DAV programmé (cas 5, étape 28), il n'existe pas de risque de fusion, et l'on peut donc choisir pour le DAV une valeur DAV-31 ms par rapport à la valeur programmée.

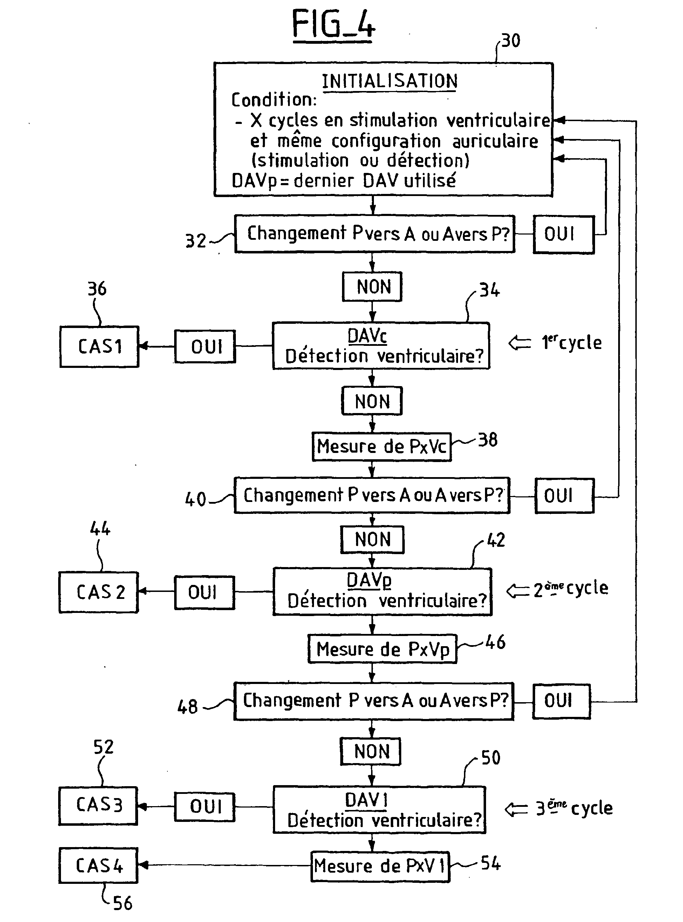

On va maintenant décrire en référence aux figures 3 et 4 la manière dont l'algorithme de détection des situations de fusion est utilisé en combinaison avec un algorithme de mesure du paramètre de capture (paramètre de détermination du seuil d'entraînement) tel que décrit par exemple dans le WO-A-93/02741 précité.

Les deux paramètres utilisés dans ce mode de mise en oeuvre sont le paramètre de capture et le délai atrio-ventriculaire.

Le DAV prendra trois valeurs différentes, au cours des trois cycles successifs illustrés sur la figure 3 :
- au premier cycle : DAV court (noté DAVc) : valeur du DAV réduit, par exemple de 31 ms, par rapport à la valeur du DAV programmé ; le DAV court peut également être réduit à une valeur fixe donnée, par exemple DAVc = 63 ms.

- deuxième cycle : DAV programmé (noté DAVp), c'est-à-dire la valeur du DAV utilisé par le stimulateur au moment du lancement de la fonction de détection de la fusion.
- troisième cycle: DAV long (noté DAVI) : valeur du DAV augmenté, par exemple de 31 ms, par rapport à la valeur du DAV programmé.

Le paramètre de capture sera noté PxVy , x étant l'amplitude de l'impulsion de stimulation (qu'il est possible de faire varier) et y étant l, c ou p, selon que la mesure est effectuée avec un DAV long, court ou programmé, respectivement.

Le déroulement de l'algorithme, illustré figure 4, est le suivant.

Au préalable (étape 30), le processus est initialisé en s'assurant qu'un certain nombre de cycles se sont succédés avec une même configuration de stimulation/détection sur les deux cavités concernées, par exemple stimulation auriculaire et stimulation ventriculaire, ou bien détection auriculaire et stimulation ventriculaire. Par ailleurs, le DAV programmé est initialisé à la valeur du dernier DAV utilisé par le stimulateur en fonctionnement normal.

L'étape suivante (étape 32) consiste à vérifier, avant toute chose, que la configuration n'a pas changé, c'est-à-dire que la stimulation auriculaire (A) n'est pas devenue une détection auriculaire (P), ou l'inverse. En cas de changement de configuration, le test est interrompu et l'algorithme retourne à l'étape d'initialisation 30, car la conduction n'étant pas la même dans les deux cas (événement auriculaire spontané ou stimulé), il n'est pas possible d'effectuer un test concluant.

Si la configuration n'a pas changé, un premier cycle est analysé (étape 34, et premier cycle de la figure 3) avec un DAV écourté, DAVc.

L'algorithme attend alors la survenue d'un événement ventriculaire spontané. Si un tel événement est détecté (étape 36), aucune mesure du paramètre de capture n'est possible, car le patient est en rythme spontané ou présente de la fusion pour la valeur du DAV programmé. L'algorithme s'achève donc avec un résultat "non concluant".

Si aucun événement ventriculaire spontané n'est détecté au cours du premier cycle, le paramètre de capture PxVc est mesuré avec ce DAV court (étape 38).

L'étape suivante (étape 40) consiste à vérifier que la configuration n'a toujours pas changé, c'est-à-dire que la stimulation auriculaire (A) n'est pas devenue une détection auriculaire (P), ou l'inverse. En cas de changement de configuration, le test est interrompu et l'algorithme retourne à l'étape d'initialisation 30, car il n'est pas possible d'effectuer un test concluant.

Si la configuration de l'oreillette n'a pas changé, alors le DAV est rétabli à sa valeur programmée DAVp et l'algorithme recherche la survenue d'un éventuel événement ventriculaire spontané au cours du cycle suivant (étape 42, et deuxième cycle de la figure 3).

Si un tel événement spontané est détecté (étape 44), il n'est pas possible de mesurer le paramètre de capture PxVp pour le DAV programmé. L'algorithme s'achève donc avec un résultat "non concluant".

Si aucun événement ventriculaire spontané n'est détecté au cours du deuxième cycle, le paramètre de capture PxVp est mesuré avec ce DAV programmé (étape 46).

Si la configuration n'a pas changé (étape 48), le DAV est allongé à la valeur DAVI et l'algorithme recherche la survenue d'un éventuel événement ventriculaire spontané au cours du cycle suivant (étape 50, et troisième cycle de la figure 3).

Si un tel événement spontané est détecté (étape 52), ceci signifie qu'il y a eu stimulation ventriculaire pour DAVc et DAVp, mais détection ventriculaire pour DAVI. Les deux valeurs PxVc et PxVp qui ont été mesurées aux étapes 38 et 46 précédente sont alors comparées. Si ces deux valeurs sont voisines, l'algorithme considère que le patient est stimulé pour la valeur du DAV programmé ; inversement, il considère que le patient est en fusion si ces deux valeurs ne sont pas voisines.

(ici et dans la suite, on entendra par "voisines" des valeurs qui sont typiquement égales entre elles à ± 20 % près, cette valeur n'étant bien entendu aucunement limitative).

Enfin, en l'absence de détection ventriculaire au troisième cycle, ceci signifie qu'il y a eu stimulation ventriculaire pour les trois cycles à DAV court, DAV programmé et DAV long. Le paramètre de capture PxVI est alors mesuré (étape 54) et les trois valeurs PxVc, PxVp et PxVI sont comparées entre elles (étape 56) :
- si les trois valeurs sont voisines (au sens indiqué plus haut) deux à deux, l'algorithme conclut à une absence de rythme spontané et de fusion ;
- si PxVc et PxVp sont voisines mais que PxVp et PxVI ne le sont pas, l'algorithme conclut à une absence de fusion au DAV programmé mais à la présence d'une fusion au DAV long ;
- si PxVp et PxVI sont voisines mais que PxVc et PxVp ne le sont pas, l'algorithme conclut à une fusion au DAV programmé au DAV long et à une capture complète au DAV court ;
- si aucune des trois valeurs PxVc, PxVp et PxVI ne sont voisines deux à deux, le signal étant très variable en fonction du DAV l'algorithme considère qu'il y a fusion pour l'ensemble des stimulations. Le résultat est "non concluant" et révèle sans doute une variabilité intrinsèque importante chez le patient.

## Revendications

1. Un dispositif médical actif implantable, notamment un stimulateur cardiaque, défibrillateur, cardioverteur ou dispositif multisite, comprenant :
- des moyens de détection d'événements auriculaire ;
- des moyens de détection d'événements ventriculaires;
- des moyens de stimulation auriculaire ;
- des moyens de stimulation ventriculaire, aptes à délivrer une impulsion de stimulation ventriculaire (V) après écoulement d'un délai atrio-ventriculaire DAV programmé consécutif à la détection d'un événement auriculaire (P, A) et en l'absence de détection d'événement ventriculaire spontané (R) dans ce délai ;
- des moyens de détection de fusion, aptes à analyser une séquence de cycles cardiaques successifs en modifiant le DAV d'un cycle à l'autre et à détecter la présence ou l'absence d'un événement ventriculaire spontané au cours d'au moins l'un des cycles de ladite séquence ; et
- des moyens pour détecter la présence ou l'absence d'un événement ventriculaire spontané survenant à l'intérieur du DAV ainsi modifié ;
dispositif **caractérisé en ce que** :
- il comprend en outre des moyens d'évaluation d'un paramètre de seuil de capture (38, 46, 54) combinant l'amplitude de l'impulsion de stimulation ventriculaire et la longueur du DAV;
- ladite séquence de cycles cardiaques successifs avec modification du DAV d'un cycles à l'autre comprend au moins trois cycles avec des DAV court, programmé et long ;
- les moyens de détection de fusion comparent deux à deux (52, 56) les valeurs (PxVc, PxVp, PxVI) du paramètre de seuil de capture au cours des cycles successifs de la séquence ;
- et il comprend des moyens pour inhiber l'évaluation du paramètre de seuil de capture en cas de détection d'un risque de fusion par les moyens de détection de fusion, l'existence d'un risque de fusion étant avéré lorsqu'au moins deux desdites valeurs sont les mêmes, à ±20% pris.

2. Le dispositif de la revendication 1, dans lequel les moyens de détection de fusion déterminent une absence de rythme spontané et de fusion lorsque les trois dites valeurs du paramètre de seuil de capture sont les mêmes, a ±20% pris.

3. Le dispositif de la revendication 1, dans lequel les moyens de détection de fusion déterminent une absence de fusion au DAV programme et une présence de fusion au DAV long, lorsque seules les deux valeurs du paramètre de seuil de capture avec des DAV court et programmé sont les mêmes, à ±20% pris.

4. Le dispositif de la revendication 1, dans lequel les moyens de détection de fusion déterminent une présence de fusion au DAV programmé et au DAV long et une capture complète au DAV court, lorsque seules les deux valeurs du paramètre de seuil de capture avec des DAV programmé et long sont les mêmes, à ±20% pris.

5. Le dispositif de la revendication 1, dans lequel les moyens de détection de fusion déterminent une présence de fusion pour l'ensemble des stimulations, lorsque qu'aucune des trois valeurs du paramètre de seuil de capture avec des DAV court, programmé et long ne sont les mêmes, à ±20% pris.

6. Le dispositif de la revendication 1, dans lequel le DAV est modifié par allongements successifs (12, 20) de sa durée (DAV, DAV+31, DAV+63).

7. Le dispositif de la revendication 1. dans lequel le DAV est modifié par raccourcissement (DAVc) et allongement (DAVI) de la durée (DAVp) du DAV programmé.

8. Le dispositif de la revendication 1, dans lequel les moyens de détection de fusion comprennent des moyens de contrôlé (32, 40, 48), à chaque cycle, de la stabilité de la configuration auriculaire, stimulée ou spontanée, et des moyens pour inhiber l'analyse de la séquence de cycles successifs en cas de changement de la configuration, de stimulée en spontanée ou l'inverse.

## Claims

1. Active implantable medical device, notably a cardiac simulator, defibrillator, cardioverter or multisite device, comprising:
- means for detecting auricular events;
- means for detecting ventricular events;
- auricular stimulation means;
- ventricular stimulation means, capable of delivering a ventricular stimulation pulse (V) after an atrioventricular delay DAV programmed following the detection of an auricular event (P, A) and, in the absence of detection of a spontaneous ventricular event (R) in this delay;
- fusion detection means, capable of analysing a sequence of successive cardiac cycles by modifying the DAV from one cycle to another and of detecting the presence or the absence of a spontaneous ventricular event during at least one of the cycles of said sequence; and
- means for detecting the presence or the absence of a spontaneous ventricular event occurring within the duly modified DAV;
a device **characterized in that**:
- it also comprises means for assessing a capture threshold parameter (38, 46, 54) combining the amplitude of the ventricular stimulation pulse and the length of the DAV;
- said sequence of successive cardiac cycles with modification of the DAV from one cycle to another comprises at least three cycles with short, programmed and long DAVs;
- fusion detection means comparing in pairs (52, 56) the values (PxVc, PxVp, PxVI) of the capture threshold parameter during successive cycles of the sequence;
- and it comprises means for inhibiting the assessment of the capture threshold parameter in case of detection of a fusion risk by the fusion detection means, the existence of a fusion risk being proven when at least two of said values are the same, to within ±20%.

2. Device according to Claim 1, in which the fusion detection means determine an absence of spontaneous rhythm and of fusion when all three said values of the capture threshold parameter are the same, to within ±20.

3. Device according to Claim 1, in which the fusion detection means determine an absence of fusion in the programmed DAV and a presence of fusion in the long DAV, when only the two values of the capture threshold parameter with short and programmed DAVs are the same, to within ±20%.

4. Device according to Claim 1, in which the fusion detection means determine a presence of fusion in the programmed DAV and in the long DAV and a complete capture in the short DAV, when only the two values of the capture threshold parameter with programmed and long DAVs are the same, to within ±20%.

5. Device according to Claim 1, in which the fusion detection means determine a presence of fusion for all the stimulations, when none of the three values of the capture threshold parameter with short, programmed and long DAVs are the same, to within ±20%.

6. Device according to Claim 1, in which the DAV is modified by successive extensions (12, 20) of its duration (DAV, DAV+31, DAV+63).

7. Device according to Claim 1, in which the DAV is modified by shortening (DAVc) and extending (DAVI) the duration (DAVp) of the programmed DAV.

8. Device according to Claim 1, in which the fusion detection means comprise checking means (32, 40, 48) for checking, in each cycle, the stability of the auricular configuration, stimulated or spontaneous, and means for inhibiting the analysis of the sequence of successive cycles in case of a change of the configuration, from stimulated to spontaneous or vice versa.

## Patentansprüche

1. Implantierbare aktive medizinische Vorrichtung, insbesondere ein Herzstimulator, -defibrillator oder -kardioverter oder eine Mehrortvorrichtung, die umfasst:
- Mittel zum Detektieren artrialer Ereignisse;
- Mittel zum Detektieren ventrikulärer Ereignisse;
- Mittel zur artrialen Stimulation;
- Mittel zur ventrikulären Stimulation, die nach Ablauf einer programmierten artrial-ventrikulären Verzögerung DAV nach der Detektion eines artrialen Ereignisses (P, A) und bei Fehlen einer Detektion eines spontanen ventrikulären Ereignisses (R) während dieser Verzögerung einen ventrikulären Stimulationsimpuls ausgeben können;
- Mittel zum Detektieren einer Fusion, die eine Sequenz aufeinander folgender Herzzyklen analysieren können und dabei die DAV von einem Zyklus zum nächsten modifizieren und das Vorhandensein oder Fehlen eines spontanen ventrikulären Ereignisses wenigstens während eines der Zyklen dieser Sequenz detektieren können; und
- Mittel, um das Vorhandensein oder Fehlen eines spontanen ventrikulären Ereignisses, das innerhalb der auf diese Weise modifizierten DAV auftritt, zu detektieren;
wobei die Vorrichtung **dadurch gekennzeichnet ist, dass**:
- sie außerdem Mittel zum Bewerten eines Einfangschwellenparameters (38, 46, 54) umfasst, die die Amplitude des Impulses der ventrikulären Stimulation und die Länge des DAV kombinieren;
- die Sequenz aufeinander folgender Herzzyklen mit Modifikation der DAV von einem Zyklus zum nächsten wenigstens drei Zyklen mit kurzer, programmierter und langer DAV enthält;
- die Mittel zum Detektieren einer Fusion die Werte (PxVc, PxVp, PxVl) des Einfangschwellenparameters während aufeinander folgender Zyklen der Sequenz paarweise vergleichen;
- und dass sie Mittel umfasst, um die Bewertung des Einfangschwellenparameters im Fall einer Detektion einer Fusionsgefahr durch die Fusionsdetektionsmittel zu verhindern, wobei die Existenz einer Fusionsgefahr gegeben ist, wenn wenigstens zwei dieser Werte bis auf +20 % gleich sind.

2. Vorrichtung nach Anspruch 1, wobei die Fusionsdetektionsmittel ein Fehlen eines spontanen Rhythmus und einer Fusion bestimmen, wenn die drei Werte des Einfangschwellenparameters bis auf ±20 % gleich sind.

3. Vorrichtung nach Anspruch 1, wobei die Fusionsdetektionsmittel ein Fehlen einer Fusion in der programmierten DAV und ein Vorhandensein einer Fusion in der langen DAV bestimmen, wenn nur die zwei Werte des Einfangschwellenparameters mit kurzer bzw. programmierter DAV bis auf ±20 % gleich sind.

4. Vorrichtung nach Anspruch 1, wobei die Fusionsdetektionsmittel das Vorhandensein einer Fusion in der programmierten DAV und in der langen DAV und einen vollständigen Einfang in der kurzen DAV bestimmen, wenn nur die zwei Werte des Einfangschwellenparameters mit der programmierten bzw. der langen DAV bis auf ±20 % gleich sind.

5. Vorrichtung nach Anspruch 1, wobei die Fusionsdetektionsmittel das Vorhandensein einer Fusion für die Gesamtheit der Stimulationen bestimmen, wenn keiner der drei Werte des Einfangschwellenparameters mit kurzer, programmierter bzw. langer DAV bis auf ±20 % gleich ist.

6. Vorrichtung nach Anspruch 1, wobei die DAV durch aufeinander folgende Verlängerungen (12, 20) ihrer Dauer (DAV, DAV + 31, DAV + 63) modifiziert wird.

7. Vorrichtung nach Anspruch 1, wobei die DAV durch Verkürzung (DAVc) und Verlängerung (DAVl) der Dauer (DAVp) der programmierten DAV modifiziert wird.

8. Vorrichtung nach Anspruch 1, wobei die Fusionsdetektionsmittel Steuermittel (32, 40, 48) umfassen, um in jedem Zyklus die Stabilität der artrialen, stimulierten oder spontanen Konfiguration zu steuern, und Mittel umfassen, um die Analyse der Sequenz aufeinander folgender Zyklen im Fall eines Wechsels der stimulierten in eine spontane Konfiguration oder umgekehrt zu verhindern.
